# EUROPEAN PATENT APPLICATION

(11) **EP 2 243 487 A1**
(43) Date of publication of application: **27.10.2010**
(21) Application number: 09100249.3
(22) Date of filing: 23.04.2009
(51) Int. Cl.: A61K 35/74, A61K 47/10, A61K 47/26, A61K 47/02, A61K 47/36, A61K 47/38, A61K 9/48

(54) **Enteric coated formulation**

(71) Applicant: Actogenix N.V., 9052 Zwijnaarde (BE)
(72) Inventor: The designation of the inventor has not yet been filed

(57) **Abstract**

The invention relates generally to the field of delayed release dosage forms for the delivery and release of beneficial and viable bacteria to the intestine or bowel. Particularly oral dosage forms containing stabilized viable bacteria using a non-hygroscopic agent and wherein said oral composition is further characterized in being stable at lower pH, in having an accelerated dissolution profile at higher pH and in being stable during long term storage.

## Description

### Field of the Invention

The invention relates generally to the field of delayed release dosage forms for the delivery of beneficial and viable bacteria to the intestine or bowel. Particularly, oral dosage forms containing viable bacteria stabilized using a non-hygroscopic agent and wherein said oral composition is further characterized in being stable at lower pH and during long-term storage, and in having an accelerated dissolution profile at higher pH.

### Background to the Invention

Live food grade bacteria, such as the lactic acid fermenting bacterial strains, *Lactococcus, Lactobacillus* or *Bifidobacterium* species, are being used in the food industry for many years because they are able to convert sugars (including lactose) and other carbohydrates into lactic acid, and are accordingly useful in the preparation of fermented dairy foods such as yogurt.

It has also been established that said lactic acid fermenting bacterial strains are beneficial to the body's naturally occurring gut flora, an ecology of microbes, and are accordingly recommended by physicians, and more frequently, by nutritionists, after a course of antibiotics, or as part of the treatment for gut related candidiasis. In these cases, the bacteria that are beneficial to our body may decrease in number, an event which allows harmful competitors to thrive, resulting in the detriment of our health. Claims are made that live food grade bacteria strengthen the immune system to combat allergies, excessive alcohol intake, stress, exposure to toxic substances, and other diseases.

More recently, recombinant strains of said food grade bacteria are also being used as promising tools in the intestinal delivery of therapeutic molecules. For example, intestinal delivery of freeze-dried, viable, recombinant hIL-10 producing *Lactococcus lactis* (Thy12) was found to have a clinical effect in patients suffering from Crohn's disease [1,2]. In the treatment of Acquired Lactase Deficiency, freeze-dried viable lactase producing bacteria, such as *Lactobacillus acidophilus,* and *Lactobacillus bulgaricus* are used in a lactase replacement therapy (US 6,008,027).

However, in spite of the usefulness and value of viable bacteria as probiotics or as intestinal delivery tools, a major restriction in the oral application of said bacteria resides in their acid-unstability. Only a trace amount of the ingested live food grade bacteria (about one per million) will survive the acidity of the gastric juice (about pH 2) and reach the intestine alive. One way to overcome this problem is by using a large excess of bacteria (10 times has been proposed in the field of food and pharmaceutical industry), however this is clearly not a fundamental solution, but merely a temporary remedy till improved formulations for the oral delivery of viable bacteria are available.

As it is also known that ambient moisture has a negative effect on the viability of freeze-dried bacteria like *Lactococcus lactis* strains, a number of enteric coated formulations have been developed to stabilise bacterial preparations for intestinal delivery.

In US 6,008,027 published 1999-12-28 the freeze-dried bacteria are mixed with a desiccant, encapsulated in an ingestible capsule, coated with an enteric polymer and treated under vacuum (from 24 to 144 hours under a vacuum of approximately 55-70 microns) to remove oxygen and moisture from the preparation. As a result of the use of drying agents, and the exposure of the capsules to vacuum pressure, the shelf life of the capsules is increased. It is however questionable whether the harsh conditions in manufacturing the enteric coated capsules, would not affect the viability of certain bacterial strains. We are accordingly of the opinion that the enteric coated formulation as described in US 6,008,027 does not provide a general solution to the intestinal delivery of viable bacteria.

In the European patents EP0828499B1 published 1998-03-18 and EP1023440B1 published 2000-08-02, microcapsules and granules containing lactic acid bacteria are coated with a water miscible coating material. Although this type of coating may be suitable for viable bacteria like *Lactobacilli,* it is known that ambient humidity in the manufacture of said coated granules has a negative effect on the viability of other bacterial strains like *Lactococcus lactis* strains.

Another major problem for the use of viable bacteria for human health applications is that international regulations require it to be stable, in particular during long-term storage, preferably up to 24 months. However, it is known in food industry that the viability of freeze dried bacteria drops seriously after freeze drying and during long term storage. Carvalho *et al*. (2003) demonstrated the stabilizing effect of either sorbitol or (mono)sodium glutamate (MSG), each added separately to lactic acid bacteria suspended in skim milk, on survival during lyophilization and subsequent storage for 3-6 months [3]. However, despite the fact that stability was increased compared to skim milk alone, the reported survival rates in the presence of sorbitol or MSG were still very low (< 0.1%). Furthermore, long-term survival of the freeze-dried cells, stored in closed containers at 20 °C in air and kept in darkness for up to 8 months, showed a significant decrease of one or more logs over time.

Huyghebaert *et al*. (2005) aimed to develop a freeze-dried powder formulation containing viable genetically modified (GM) L. lactis bacteria with an acceptable shelf life, suited for ileal targeting [4]. They developed enteric-coated capsules (HPMC) caps, and performed short- and long-term stability studies. However, even though capsules were filled in a production room with low relative humidity (20% RH) and the powder was kept on ice, viability already decreased with > 30% during manipulation. Further, long-term stability results showed that, after storage for 1 year at 8°C and -20°C respectively, only 6 and 44% of the initial cell count remained viable. Finally, experiments focusing on viability and maintenance of GM properties after passage through gastric fluid, showed that viability of the GM L. lactis bacteria in the enteric-coated capsules significantly decreased after subjecting them for 2 hours to 0.1 N HCl (< 40% remained viable in Eudragit FS 30 D coated capsules, and < 30% in Eudragit L30D-55 coated capsules).

Given the shortcomings of the currently available formulations for viable bacteria, it is an object of the present invention to provide a general solution to the intestinal delivery of viable bacteria, including the delivery of the more sensitive bacteria like *Lactococcus lactis* strains.

Compared to the aforementioned references and in particular US 6,008,027, the enteric-coated formulations of the present invention differ in the application of a non-hygroscopic excipient to stabilize the viable bacteria. As explained in more detail in the examples hereinafter, notwithstanding the observed infiltration of gastric juice in the capsules of the present invention, and a change in the aggregation status of the content of the capsules, the presence of the non-hygroscopic excipient was found to buffer (prevent) an increase of the moisture content of the capsules and accordingly contributes to the stabilization and viability of the bacteria upon intestinal delivery.

This and other aspects of the present invention will be explained in more detail hereinafter.

### References:

1. Steidler L, Neirynck S, Huyghebaert N, Snoeck V, Vermeire A, Goddeeris B, Cox E, Remon JP, Remaut E. Biological containment of genetically modified Lactococcus lactis for intestinal delivery of human interleukin 10. Nat Biotechnol 2003;21:785-789
2. Braat H, Rottiers P, Hommes DW, Huyghebaert N, Remaut E, Remon JP, van Deventer SJ, Neirynck S, Peppelenbosch MP, Steidler L. A phase I trial with transgenic bacteria expressing Interleukin-10 in Crohn's disease. Clinical Gastroenterology and Hepatology 2006;4:754-759
3. Carvalho A, Silva J, Ho P, Teixeira P, Malcata F, Gibbs P. Protective effect of sorbitol and monosodium glutamate during storage of freeze-dried lactic acid bacteria. In; 2003:203-210
4. Huyghebaert N, Vermeire A, Neirynck S, Steidler L, Remaut E, Remon JP. Development of an enteric-coated formulation containing freeze-dried, viable recombinant Lactococcus lactis for the ileal mucosal delivery of human interleukin-10. European Journal of Pharmaceutics and Biopharmaceutics 2005;60:349-359

### Brief Description of the Drawings

### Figure 1

Moisture levels as measured by Karl-Fischer titration (% water) on different samples during placebo capsule production: the placebo freeze-dried cake(s), the two fillers (mannitol and anhydrous dicalciumphosphate), the powder mixes of placebo cake with fillers, the capsule powder content after filling, as well as the powder content and capsule shell after banding and after coating of capsules produced with the two fillers (mannitol and anhydrous dicalciumphosphate).

### Figure 2

Moisture levels as measured by Karl-Fischer titration (% water) on different samples during active capsule production (containing live recombinant *L.lactis*): the active freeze-dried cake(s), the two fillers (mannitol and anhydrous dicalciumphosphate), the powder mixes of placebo cake with fillers, the capsule powder content after filling, as well as the powder content and capsule shell after banding and after coating of capsules produced with the two fillers (mannitol and anhydrous dicalciumphosphate).

### Figure 3

Moisture levels as measured by the Loss on Drying method (LOD) (% water) during challenge testing on different samples of active capsules (containing live recombinant *L.lactis*) with the two fillers (mannitol and anhydrous dicalciumphosphate). The moisture levels are presented on capsule powder content and capsule shell before and after challenge testing in different media: for 2 hours in 0.1N HCl (pH 1), which resembles gastric juice conditions, and subsequently for 1 hour in phosphate buffer(pH 5.5), which mimics the pH in the duodenum.

### Figure 4

Viability of L.lactis strain sAGX0037 bacteria, as measured by viable cell count (CFU/g) on different samples during active capsule production (containing live recombinant *L.lactis sAGX0037*): the powder mixes of freeze-dried active cake with fillers (Drug Product DP) after mixing, after filling and after banding/coating (2 Eudragit L-100 polymer coating levels : 12.5 and 15 mg/cm²) of capsules produced with the two fillers (mannitol and anhydrous dicalciumphosphate).

### Figure 5

Viability of L.lactis strain sAGX0037 bacteria, as measured by viable cell count (CFU/g) during challenge testing on different samples of Eudragit L-100 coated capsules (containing live recombinant *L.lactis*) formulated with anhydrous dicalciumphosphate. The viable cell count is presented on capsule powder content, challenged in different media: for 0, 1 and 2 hours in 0.1N HCl (pH 1), which resembles gastric juice conditions and for 0, 1 and 2 hours in water (H2O).

### Detailed Description of the Invention

In a first embodiment, the present invention provides an enteric coated capsule comprising stabilized freeze-dried viable bacteria, in particular recombinant bacteria, **characterized in that** the viable recombinant bacteria are stabilized using a non-hygroscopic agent. As explained in more detail in the examples hereinafter, notwithstanding the observed infiltration of gastric juice upon 2 hours exposure in 0.1N HCl and 1 additional hour at a pH of 5.5, it was surprisingly found that the viability was not affected for the stabilized cells of the present invention.

As used herein a non-hygroscopic agent is meant to include any excipient typically used in the formulation of a pharmaceutical composition and wherein said agent exhibits an equilibrium moisture uptake at ambient 40% RH of not more than about 8%, preferably not more than about 7%, and more preferably not more than about 6%, for example about 1% to about 5%, more in particular less or equal to 2%. The non-hygroscopic agent can be a polyol such as for example mannitol, maltitol, isomalt (polyol sugar) or a phosphate salt such as for example anhydrous dicalcium phosphate dibasic calcium phosphate, calcium hydrogen phosphate, or for example a sugar such as sucrose.

The capsule used in the aforementioned formulation is typically selected from the group consisting of a gelatin capsule, a starch capsule, a hydroxypropylmethylcellulose (HPMC) capsule and the like; in particular a HPMC capsule. Compared to gelatin capsules the latter have better coating properties (the smooth surface of the gelatin capsules can lead to shell embritlement and poor adhesion of the enteric coating), and can be filled using standard and widely available capsule filling machines. In this respect it was additionally found that the presence of the non-hygroscopic agent (stabilizer) improved the flow properties during automatic filling of the HPMC capsules.

For the intestinal delivery of viable bacteria, the enteric-coated capsules of the present invention should be stable at low pH (up to pH 5.5) and have an accelerated dissolution profile at higher pH (above pH 5.5). The optimal release is realized when the capsules desintegrate at a pH of about 6.8 within 1 hour. Thus, in a further aspect of the present invention the capsules are coated with an enteric polymer to provide an enteric coated capsule that is stable at a pH up to 5.5 and that is soluble at a pH above 5.5; in particular at a pH above 6.0; more in particular with a fast dissolution profile at a pH of about 6.8.

The enteric polymer used for the enteric coating typically consists of a film-formable polymeric substance, which is soluble at a pH above 5.5, in particular at a pH above 6.0.

Film-formable polymers useful in the different embodiments of the present invention are usually selected from the group consisting of a cellulose derivative, an acrylic copolymer, a maleic copolymer, a polyvinyl derivative, shellac and the like; in particular an acrylic copolymer selected from the group consisting of styrene-acrylic acid copolymer, methyl acrylate-acrylic acid copolymer, methyl acrylate-methacrylic acid copolymer, butyl acrylate-styrene-acrylic acid copolymer, methacrylic acid-methyl methacrylate copolymer such as Eudragit L100, Eudragit S or Eudragit S100 (each being trade name, commercially available from Röhm Pharma, Germany), methacrylic acid-ethyl acrylate copolymer such as Eudragit L100-55 (trade name, commercially available from Röhm Pharma, Germany), methyl acrylate-methacrylic acid-octyl acrylate copolymer, and the like; more in particular the film-formable polymer consists of methacrylic acid-methyl methacrylate copolymer.

In a further embodiment of the present invention, the capsules are sealed after filling in the overlapping region of capsule and cap by commonly known sealing techniques like banding or applying a sealing and/or heat to the gap between capsule body and cap. Preferred is a banding in which a banding solution, which may include a solvent, is applied individually and uniformly to the external edge of the gap of a capsule to be sealed to form a liquid ring around the circumference of the capsule. Removing excess banding solution from the exterior of the capsule and drying the banding before coating will prevent problems e. g. with nonuniformity of the coating at the gap or development of fissures during storage under stressing conditions, which can lead to an unwanted loss of gastro resistant properties.

The enteric-coated capsules of the present invention are particularly useful for the intestinal delivery of freeze dried viable bacteria, including recombinant bacteria capable of expressing one or more biologically active polypeptides, in particular lactic acid fermenting bacterial strains, selected from the group consisting of *Streptococcus, Lactococcus, Lactobacillus* or *Bifidobacterium;* more in particular *Lactococcus lactis*.

Surprisingly, the enteric-coated capsules of the present invention, comprising the non-hygroscopic agents, are extremely beneficial for the maintenance of the viability of freeze dried bacteria after encapsulation and during storage of primary packed enteric coated capsules at different temperatures. More than 50%, preferably more than 70%, more preferably more than 90% of the initial viability of the bacteria after the capsule production process is maintained during 12 months of storage at 2-8°C. In view of the fact that it is known that stability at room temperature is difficult to obtain, it was surprisingly found that more than 50%, preferably more than 70%, more preferably more than 80% of the initial viability of the bacteria after the capsule production process is maintained during 6 months storage at room temperature (25°C).

As already mentioned hereinbefore, the presence of the non-hygroscopic agent also improves the flow properties of the powder mixture used in filling the capsules. Further addition of a lubricant like talc, further improves the filling efficiency of said mixture in avoiding adhesion of the powder mixture to the surface of the punches in the capsule filling machines. Thus in a particular embodiment of the enteric coated capsules of the present invention, they further comprise up to 5.0 % (w/w) of a lubricant like talc, which is also a non-hygroscopic agent.

It is also an object of the present invention, to provide a process to prepare enteric coated capsules as defined herein, said method comprising the steps of;
- mixing freeze dried viable recombinant bacteria with the lubricant and a non-hygroscopic agent to produce stabilized freeze dried viable recombinant bacteria;
- encapsulating a unit dosage amount of said stabilized freeze dried viable recombinant bacteria in a human or animal ingestible capsule;
- optionally sealing said capsule with a banding solution; and
- coating said sealed capsule with an enteric polymer.

This invention will be better understood by reference to the Experimental Details that follow, but those skilled in the art will readily appreciate that these are only illustrative of the invention as described more fully in the claims that follow thereafter. Additionally, throughout this application, various publications are cited. The disclosure of these publications is hereby incorporated by reference into this application to describe more fully the state of the art to which this invention pertains.

### EXAMPLES

The following examples illustrate the invention. Other embodiments will occur to the person skilled in the art in light of these examples.

The main purpose of the formulation and manufacturing process development was to provide protection of highly sensitive freeze dried bacteria in enteric-coated capsules from moisture

### EXAMPLE 1: Selection of the excipients

To provide protection of bacteria in enteric coated capsules against moisture, two excipients (fillers) were selected based on their non-hygroscopicity and low moisture content, however, other non-hygroscopic excipients could be used as well, as described above.
1. **Mannitol** has a moisture content less than 0.5% and is widely used in the formulation of solid dosage forms as a diluent. It is of particular value since it is non-hygroscopic and may thus be used with moisture-sensitive active ingredients (Allan LV, International Journal of Pharmacological Compounds, 2000; 4(4): 306-310, 324-325).
2. **Anhydrous dicalcium phosphate** has a moisture content less than 2.0% and is non-hygroscopic. It is of particular interest in capsule formulations because of its compaction properties, good flow properties and high density (Fischer E, Manuf Chemist, 1992; 64(6): 25-27).

### EXAMPLE 2: Production process of placebo capsules

Both fillers were used in an experiment to determine whether they are useful for high-throughput production of enteric coated capsules. Therefore, a placebo freeze-dried cake was produced with the same composition as the active cake, however lacking the viable bacteria.

### 1. Mixing

This placebo freeze-dried cake (150 mg/capsule) was blended for 3 minutes at 1500 - 1800 rpm with a Stephan UMC 5 blender to obtain a flowable powder. Next, the filler (mannitol or anhydrous dicalcium phosphate) (150 mg/capsule) was added to the blender and mixed with the powder of the placebo cake for another 3 minutes at 600 rpm impeller speed. This mixture was used during an automated encapsulation process for filling HPMC capsules of size 1.

### 2. Filling

Anhydrous dicalcium phosphate scored better during the automated encapsulation process compared to mannitol. Mannitol tends to produce material build-up on the wall of the filling dies. Therefore, capsules could often not be ejected properly, leading to increased ejection forces, which leads to damage of the capsule shell.

### 3. Optional Banding

After filling of the capsules, the gap between both halves of the capsule was closed by banding using standard procedures. The banding was performed at room temperature using the Bonapace BD3000 equipment according to the batch formula in table 1. From time to time, the banding solution was stirred to avoid settling of the suspension. Fresh suspension was added periodically to the suspension vessel. Other banding solutions or immediate enteric coating without prior banding can also be used.

**Table 1. Batch formula for the used banding solution**

| **Component** | **% w/v** | **Quantity per unit** |
|---|---|---|
| HPMC Hypromellose 603 | 10% | 2 mg |
| Ethanol 96% | 50% | |
| Microcrystalline cellulose | 12% | 2.4 mg |
| Glycerol 85 % | 2% | 0.4 mg |
| Acetone | 26% | |

### 4. Enteric coating

After banding, the capsules were coated with an Eudragit^{®} L100 coating solution as further specified in table 2. Other standard enteric coating solutions as described above, can also be used.

**Table 2. Batch formula for the Eudragit^{®} L100 coating solution**

| **Component** | **% w/w** |
|---|---|
| Eudragit^{®} L100: Methacrylic acid - methyl methacrylate copolymer (1:1) | 8.3% |
| PEG 20.000 | 1.7% |
| Isopropanol | 85,8% |
| Purified water | 4,2% |

The film coating was performed using the pan coating equipment according to the batch formula indicated in table 2 and the coating parameters as indicated in table 3. Purified water was dosed into a vessel and PEG 20.000 was added. Propeller stirrer was used to mix the solution until a clear solution of Macrogol in water was obtained. Isopropanol was dosed into a separate container and Eudragit^{®} L100 was added whilst stirring at 1100-1200 rpm with a propeller stirrer for 2 hours. Additional amount of water was added to the dispersion and the Macrogol dispersion was added to the Eudragit^{®} L100 dispersion whilst stirring at 1100-1200 rpm. The prepared dispersion was continuously stirred at 100-400 rpm during the coating process to prevent sedimentation of Macrogol. The amount of Eudragit^{®} L100 in the batch formula corresponds to a theoretical coating density of 20 mg/cm², however, the coating process was stopped after a coating density of 12.5 mg/cm², which is sufficient to obtain gastro-resistant properties.

**Table 3. Coating parameters for placebo capsules**

| **Coating Parameter** | **Value** |
|---|---|
| Inclination of coating pan | 60° |
| Rotational speed (rpm) | 15 |
| Nozzle diameter (mm) | 1.0 |
| Atomising pressure (bar) | 2.5 |
| Spraying rate (g/min) | 12 - 18 |
| Inlet air temperature (°C) | 41.2 - 56.3 |
| Exhaust air temperature (°C) | 23.2 - 25.7 |
| Product temperature (°C) | 26.2 - 31.3 |
| Drying temperature (°C) | 40 |

### Example 3. Analysis of moisture content of placebo capsules

The placebo capsules as produced according to example 2, were used to determine the effect of anhydrous dicalcium phosphate and mannitol on the moisture levels of the capsule shell as well as on the powder content of the capsules. Moisture of capsule shell and capsule content was measured by the Karl Fischer (KF) method according to Ph. Eur. Method 2.5.32. Therefore, 100 to 500 mg of product was uniformly grinded in a mortar (provided that the capsule shell was analyzed) or poured directly into the titration vessel (provided that the capsule content was analyzed). Hydranal 5 (solution of imidazole, sulphur oxide and iodine in diethylene glycol monoethyl ether) was used as titration solution.

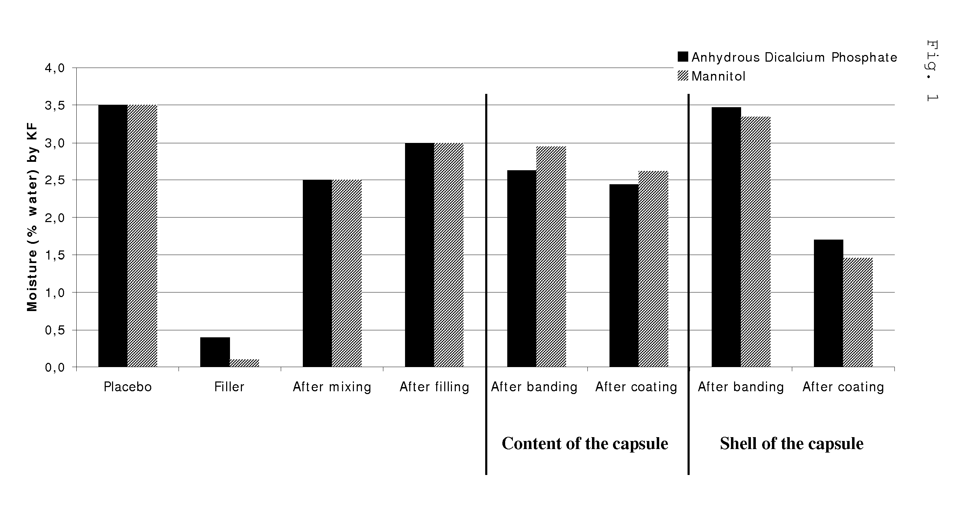

The moisture content was determined at the following steps during the production process (figure 1):
- Before mixing → Placebo cake
   → Filler
- After mixing (placebo cake with filler)
- After filling of the capsules
- After banding (content (placebo cake with filler) as well as shell)
- After coating (content (placebo cake with filler) as well as shell)

As shown in figure 1, both fillers have a very low moisture content. Therefore, after mixing with the placebo cake, the moisture values drop compared to the placebo cake before mixing. Although there is a slight increase in moisture values of the capsule content after filling of the capsules, these values decrease after banding and subsequent coating of the capsules for both fillers. However, at all steps during production of the capsules, the moisture content of the cake/filler mix remains below the initial moisture content of the placebo cake, indicating that both fillers prevent moisture uptake during capsule production.

### EXAMPLE 4: Production process of capsules containing live recombinant L.lactis

Freeze dried, viable *lactococcus lactis* bacteria were used to produce active capsules containing anhydrous dicalcium phosphate or mannitol with a process very similar to the one described for placebo capsules (example 2).

### 1. Mixing

The active cake (150 mg/capsule) was blended for 3 minutes at 1500 - 1800 rpm with a Stephan UMC 5 blender to obtain a flowable powder. Additionally talcum (15 mg/capsule) was added as a lubricant to improve the lubrication during the filling process. Next, the filler (mannitol or anhydrous dicalcium phosphate) (150 mg/capsule) was added to the blender and mixed with the powder of the active cake for another 3 minutes at 600 rpm impeller speed. This mixture was used during an automated encapsulation process for filling of HPMC capsules of size 1.

### 2. Filling

Anhydrous dicalcium phosphate scored much better during the automated encapsulation process compared to mannitol. The mannitol/talcum mixture, mixed with the active cake tended to be even stickier than the placebo cake mixed with mannitol. The yield of capsules with anhydrous dicalcium phosphate was more than 80% in two separate clinical batches at batch scale of 2000 and 9000 capsules, respectively. (see table 4).

**Table 4. Yield of capsules after production process with anhydrous dicalcium phosphate as filler**

| | **Placebo Cake** | **1.2x10E+10 CFU/caps** | **1.2x10E+11 CFU/caps** |
|---|---|---|---|
| **Step 1 Mixing** | | | |
| Nominal amount | 3,72 kg | 0,6212 | 2,79 |
| Actual yield | 3,68 kg | 0,62 | 2,75 |
| Yield (%) | 99 | 100 | 99 |
| Moisture KF (%) | 0,4 | 0,8 | 2,8 |
| **Step 2 Filling and** | | | |
| **banding** | | | |
| Nominal amount | | | |
| (pcs.) | 12.000 | 2.004 | 9.004 |
| Actual yield | 11.820 | 1.811 | 7.919 |
| Yield (%) | 98,5 | 90,4 | 87,9 |
| Moisture KF (%) | 0,4 | 0,6 | 2,8 |
| **Step 3 Coating** | | | |
| Nominal amount | | | |
| (pcs.) | 11.820 | 1.761 | 7.869 |
| Actual yield (pcs.) | 11.552 | 1.680 | 7.812 |
| Yield (%) | 97,7 | 95,4 | 99,3 |
| Moisture KF (%) | 0,3 | 0,5 | 2,7 |
| **Overall yield** | | **83,83** | **86,76** |

### 3. Optional Banding

After filling of the capsules, the gap between both halves of the capsule was closed by banding using standard procedures. The banding was performed at room temperature using the Bonapace BD3000 equipment according to the batch formula in table 1. From time to time, the banding solution was stirred to avoid settling of the suspension. Fresh suspension was added periodically to the suspension vessel. Other banding solutions or immediate enteric coating without prior banding can also be used.

### 4. Enteric coating

After banding, the capsules were coated with an Eudragit^{®} L100 coating solution as further specified in table 2. Other standard enteric coating solutions as described above, can also be used.

The film coating was performed using the pan coating equipment according to the batch formula indicated in table 2 and the coating parameters as indicated in table 5. Purified water was dosed into a vessel and PEG 20.000 was added. Propeller stirrer was used to mix the solution until a clear solution of Macrogol in water was obtained. Isopropanol dosed into a separate container and Eudragit^{®} L100 was added whilst stirring at 1100-1200 rpm with a propeller stirrer for 2 hours. Additional amount of water was added to the dispersion and the Macrogol dispersion was added to the Eudragit^{®} L100 dispersion whilst stirring at 1100-1200 rpm. The prepared dispersion was continuously stirred at 100-400 rpm during the coating process to prevent sedimentation of Macrogol. The amount of Eudragit^{®} L100 in the batch formula corresponds to a theoretical coating thickness of 20 mg/cm², however, the coating process was stopped after a coating thickness of 12,5 or 15 mg/cm², which is sufficient to obtain gastro-resistant properties.

**Table 5. Coating parameters for active capsules**

| **Coating Parameter** | **Value** |
|---|---|
| Inclination of coating pan | 60° |
| Rotational speed (rpm) | 15 |
| Nozzle diameter (mm) | 1.0 |
| Atomising pressure (bar) | 2.5 |
| Spraying rate (g/min) | 22 - 26 |
| Inlet air temperature (°C) | 49.6 - 55.9 |
| Exhaust air temperature (°C) | 21.4 - 24.7 |
| Product temperature (°C) | 27.2 - 31.9 |
| Drying temperature (°C) | 40 |

### Example 5. Analysis of moisture content in active capsules

The active capsules as produced according to example 4, were used to determine the effect of anhydrous dicalcium phosphate and mannitol on the moisture content of the capsule shell as well as on the content of the capsules. Moisture of capsule shell and capsule content was measured by the Karl Fischer (KF) method according to Ph. Eur. Method 2.5.32. Therefore, 100 to 500 mg of product was uniformly grinded in a mortar (provided that the capsule shell was analyzed) or poured directly into the titration vessel (provided that the capsule content was analyzed). Hydranal 5 (solution of imidazole, sulphur oxide and iodine in diethylene glycol monoethyl ether) was used as titration solution.

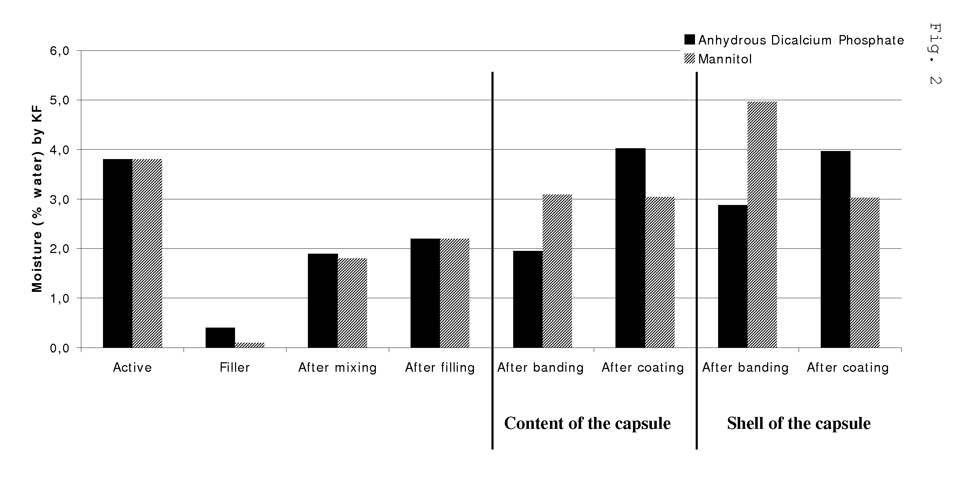

The moisture content was determined at the following steps during the production process (figure 2):
- Before mixing → Active freeze dried cake
   → Filler
- After mixing (active freeze dried cake with filler)
- After filling of the capsules
- After banding (content (active cake with filler) as well as shell)
- After coating (content (active cake with filler) as well as shell)

As shown in figure 2, both fillers score equally well on the moisture content test during mixing of the compounds and filling of the capsules. Also during capsule banding and subsequent coating, the use of both fillers protects the freeze dried viable bacteria content from moisture uptake and exposure during production. The observed differences in moisture values of the capsule powder content between Mannitol and anhydrous dicalcium phosphate is within the analytical variation of the KF methodology used.

In conclusion, for both mannitol and anhydrous dicalcium phosphate the moisture values of the capsule content at the end of the production only slightly increase or even decrease when compared to the moisture content of the active ingredient (the powder of viable bacteria) at the start of the procedure. Accordingly both fillers protect the freeze dried viable bacteria from moisture exposure during production of the enteric coated capsules of the present invention, without the need of extensive drying steps like the vacuum exposure in US 6,008,027.

Since enteric coated capsules, and in particular the freeze dried viable bacteria contained therein, need to withstand harsh conditions in the stomach, such as gastric juice, and pH conditions of the proximal part of the small intestine (duodenum), before they can deliver their content in the distal part of the small intestine (ileum) and lower down the gastrointestinal tract, the effect of these conditions on the moisture content was analyzed on the active capsules. Therefore the active capsules were challenged in different media: for 2 hours in 0.1N HCl (pH 1), which resemble gastric juice conditions, and subsequently for 1 hour in phosphate buffer(pH 5.5), which mimics the pH in the duodenum. The moisture content before and after said challenges was determined by assessing the loss on drying (LOD) using an infrared balance. Thereto, 500 to 1500 mg of product was evenly distributed on the measurement plate. Temperature of the infrared lamp was adjusted to 105°C and the measurement was performed for 8 minutes.

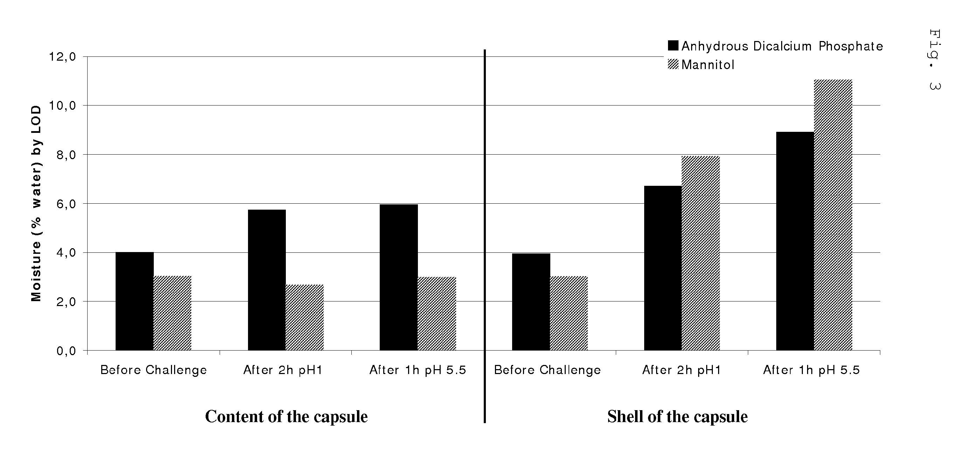

When evaluating the aspect of the content of the capsules after the aforementioned challenge, a change in structure was observed. The capsule filling mass is no longer a free flowing powder but a gelly stucture instead. Apparently there is infiltration of test medium, representing 'gastric juice' into the capsules, which is absorbed, in the capsule content. As shown in figure 3, however, although the moisture values for the shell more than double during challenge with 0.1N HCl for 2 hours and subsequently for 1 hour in phosphate buffer, the moisture values for the capsule powder content are clearly not or only mildly affected. For Mannitol there is hardly any change in the moisture level of the capsules powder content, and for Anhydrous dicalcium phosphate the increase in moisture plateaus at about 6,0%. Only a maximum increase with 1.8 % moisture of the capsule powder content is detected during this 3 h challenge test.

### Example 6. Analysis of viability of bacteria in active capsules during production and challenge

The viability of the Lactococcus Lactis bacteria inside the capsule during production and subsequent challenge at different pH values was analyzed by plating them on GM17T agar plates (M17 broth, supplemented with 0,5% glucose and 200 µm thymidine). The plates were incubated for 16 - 24 hours at 30°C and the number of colonies per plate was counted. The average number of CFU/g was calculated.

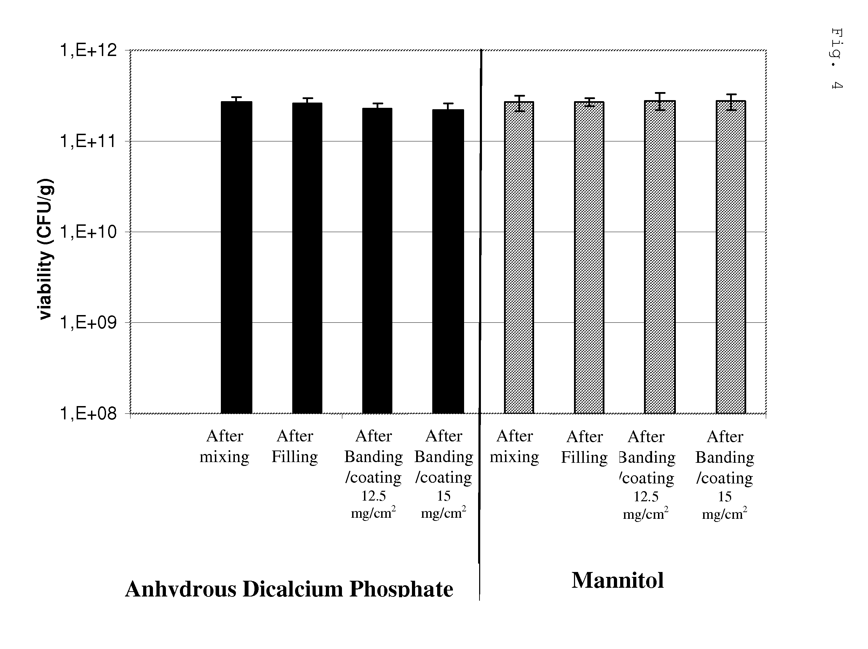

As shown in figure 4, both fillers protect the viability of the bacteria equally well after mixing with the filler, filling of the capsules and subsequent banding and coating of the capsules. A high viable cell yield of > 2 x 10E+11 CFU/g is maintained during the production of the capsules, for both fillers tested.

Since both mannitol and anhydrous dicalcium phosphate scored equally well for the cell viability testing during production of the capsules, but the yield of capsules during production was much higher for anhydrous dicalcium phosphate (see example 2), capsules filled with this latter excipient were further used for testing viability after subsequent challenge.

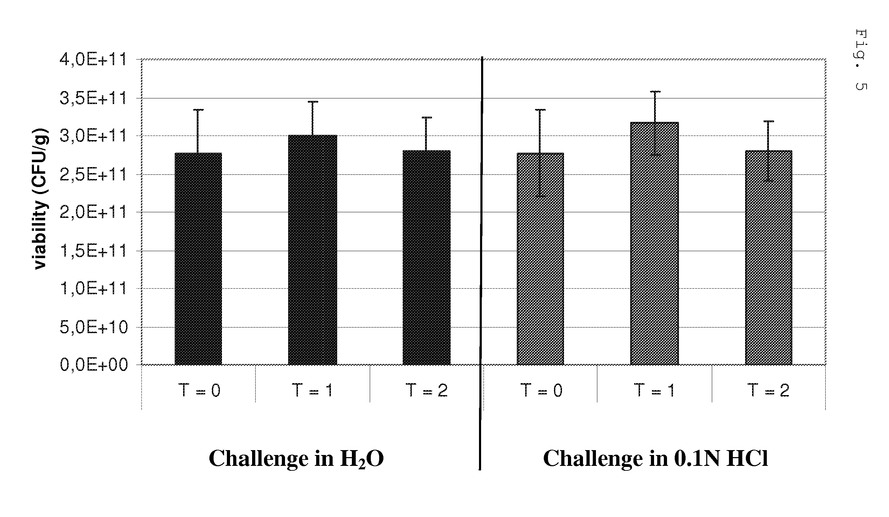

As shown in figure 5, even during the challenge with either water or HCl 0.1N pH 1 (2h), cell viability of powder content is clearly not affected and therefore anhydrous dicalcium phosphate protects the viability of the bacteria when the capsules are challenged in a medium which resembles gastric juice conditions (eg. in 0.1N HCl (pH 1)) or in water.

### Example 7.Analysis of viability of bacteria after long term storage.

As shown in table 6, the viability of the freeze dried bacteria, stabilized with anhydrous dicalcium phosphate; is maintained after encapsulation and during storage of primary packed enteric coated capsules at different temperatures (-20°C, 5°C, 25°C). More than 90% of the initial viability of the bacteria after the capsule production process is maintained during 12 months of storage at 2-8°C, and more important, more that 80% of the initial viability of the bacteria is maintained at 6 months storage at room temperature (25°C). These data indicate that a non-hygroscopic excipient such as anhydrous dicalcium phosphate is not only useful for maintaining viability of freeze dried bacteria during production and challenge of enteric coated capsules, but also for stabilizing survival of the bacteria during long-tem storage of the capsules.

**Table 6. Average viable cell count after storage**

| | **Condition** | | |
|---|---|---|---|
| | **-20°C** | **5 °C** | **25°C** |
| **Time (months)** | CFU/g | CFU/g | CFU/g |
| 0 | | | 3,20E+ 11 |
| 1 | 3,46E+ 11 | 3,84E+ 11 | 3,14E+ 11 |
| 2 | 3,84E+ 11 | 3,42E+ 11 | 2,64E+ 11 |
| 3 | 3,48E+ 11 | 3,23E+ 11 | 2,46E+ 11 |
| 6 | 3,39E+ 11 | 3,27E+ 11 | 2,68E+ 11 |
| 9 | 3,27E+ 11 | 3,28E+ 11 | |
| 12 | 2,90E+ 11 | 3,20E+ 11 | |

## Claims

1. An enteric-coated capsule comprising stabilized freeze dried viable bacteria, **characterized in that** the viable bacteria are stabilized using a non-hygroscopic agent.

2. The enteric-coated capsule according to claim 1 wherein the non-hygroscopic agent is selected from a polyol or a phosphate salt or a sugar, or any combination thereof.

3. The enteric-coated capsule according to claim 2 wherein the polyol is either mannitol, maltitol or isomalt, or combinations thereof.

4. The enteric-coated capsule according to claim 2 wherein the phosphate salt is either dicalcium phosphate dibasic calcium phosphate or calcium hydrogen phosphate in hydrate or anhydrous form, or combinations thereof.

5. The enteric-coated capsule according to claim 2 wherein the sugar is sucrose.

6. The enteric-coated capsule according to any of the claims 1 to 5, wherein the freeze dried viable bacteria are lactic acid fermenting bacterial strains, selected from the group consisting of *Streptococcus, Lactococcus, Lactobacillus* or *Bifidobacterium;* more in particular *lactococcus lactis.*

7. The enteric-coated capsule according to any of the claims 1 to 6, wherein the freeze died viable bacteria are recombinant bacteria, capable of expressing one or more biologically active polypeptides.

8. The enteric coated capsule according to any of the claims 1 to 7 wherein the capsule is selected from the group consisting of a gelatin capsule, a starch capsule, a hydroxypropylmethylcellulose (HPMC) capsule and the like; in particular a HPMC capsule.

9. The enteric coated capsule according to any of the claims 1 to 8 wherein the capsule is coated with a film-formable polymeric substance which is soluble at a pH above 5.5.

10. The enteric coated capsule according to any of the claims 1 to 9, wherein the coating further comprises an emulsifier and an anti-foaming agent.

11. The enteric coated capsule according to any of the claims 1 to 10, further **characterized in that** it is sealed with a banding solution.

12. The enteric-coated capsule according to any of the claims 1 to 11, further comprising up to 5.0 % (w/w) of a lubricant like talc, magnesium stearate, and glycerol monostearate.

13. The enteric-coated capsule according to any of the claims 1 to 12 wherein the enteric coating is applied in the range of 10 - 20 mg/cm² of capsule surface.

14. A process to prepare enteric coated capsules as defined in any one of claims 1 to 13, comprising the steps of;
- mixing freeze dried viable recombinant bacteria with a non-hygroscopic agent to produce stabilized dried viable recombinant bacteria;
- encapsulating a unit dosage amount of said stabilized freeze dried viable recombinant bacteria in a human or animal ingestible capsule;
- optionally sealing said capsule with a banding solution; and
- coating said sealed capsule with an enteric polymer.
